# EUROPEAN PATENT SPECIFICATION

(11) **EP 2 393 448 B1**
(45) Date of publication and mention of the grant of the patent: **25.06.2014**
(21) Application number: 10704492.7
(22) Date of filing: 04.02.2010
(51) Int. Cl.: A61B 17/04, A61B 17/06, A61F 2/00

(54) **IMPLANTABLE DEVICES AND TOOLS FOR ANATOMICAL SUPPORT**
IMPLANTIERBARE VORRICHTUNGEN UND WERKZEUGE FÜR DIE ANATOMISCHE UNTERSTÜTZUNG
DISPOSITIFS IMPLANTABLES ET OUTILS POUR SUPPORT ANATOMIQUE

(30) Priority: 05.02.2009 US 150276 P; 31.03.2009 US 414709; 19.11.2009 US 621517
(43) Date of publication of application: 14.12.2011
(73) Proprietor: Coloplast A/S, 3050 Humlebæk (DK)
(72) Inventor: MORNINGSTAR, Randy L., Brooklyn Park, Minnesota 55444 (US); WITZMANN, Michael M., Minneapolis, Minnesota 55418 (US); MOSCHEL, Mark A., New Hope, Minnesota 55427 (US)
(86) International application number: PCT/DK2010/050035
(87) International publication number: WO 2010/088917

(56) References cited:
- WO-A1-2005/122954
- WO-A1-2006/108145
- WO-A2-2007/059199
- WO-A2-2007/149555
- US-A1- 2005 004 576

## Description

### TECHNICAL FIELD

This disclosure relates generally to medical devices. More particularly, this disclosure relates to implantable devices, tools, and methods for anatomical support.

### BACKGROUND

Devices for anatomical support, and particularly those for treatment of urinary incontinence and pelvic organ prolapse have been proposed in recent years. Such devices have included suburethral sling devices for urinary incontinence, and mesh devices for pelvic organ prolapse. Sling devices are surgically implanted under a patient's urethra to provide support to the urethra so that during a provocative event such as coughing or laughing, urine is inhibited from leaking out of the urethra. Devices for treatment of pelvic organ prolapse are also surgically implanted, to inhibit herniation or prolapse of an organ (e.g., the bladder) into the vaginal space. Such support from the sling and mesh devices replaces natural anatomical support that is lacking in the patient. But implanting and anatomically securing some devices may be difficult and time consuming. Further, in the case of urinary incontinence, some sling devices may provide unreliable anatomical fixation and unacceptable adjustment or tensioning for supporting the urethra, thereby leading to suboptimal or even unacceptable results for treatment of urinary incontinence.

US2005/0004576 discloses self-anchoring slings and deployment mechanisms for use therewith in selectively positioning a sling into position within the body. According to a preferred embodiment, the sling comprises an elongate sling portion having opposed ends. Formed upon each respective opposed end is an anchor member operative to be percutaneously advanced through soft tissue at a selected target site in a first direction but resist movement in an opposed direction.

### SUMMARY

This disclosure describes novel implantable devices that provide support to a urethra or other anatomical structure. This disclosure also describes novel tools with the implantable devices, as defined in the appended claims.

In one aspect, an implantable device for anatomical support includes a sling, a first interconnecting member that is coupled to the sling, and a second interconnecting member that is coupled to the sling. An adjustable anchor is slidably coupled to the first interconnecting member to permit bi-directional movement along the first interconnecting member, and configured to exert a compressive force generating frictional interference between the adjustable anchor and the first interconnecting member, to inhibit the bi-directional movement of the adjustable anchor along the first interconnecting member unless sufficient force is applied to overcome the frictional interference. Also, a fixed anchor is fixedly coupled to the second interconnecting member. In another aspect, the first interconnecting member and the second interconnecting member are sutures. In another aspect, the first interconnecting member and the second interconnecting member are materials having an overall width approximating that of a surgical suture.

In another aspect, an implantable device for anatomical support includes a sling, a first interconnecting member that is coupled to the sling, and a second interconnecting member that is coupled to the sling. An anchor is provided in freely sliding engagement with the first interconnecting member. A tensioning element is slidably coupled to the first interconnecting member to permit movement along the first interconnecting member and configured to exert a compressive force generating frictional interference between the tensioning element and the first interconnecting member, to inhibit the movement of the tensioning element along the first interconnecting member unless sufficient force is applied to overcome the frictional interference. Also, a fixed anchor is fixedly coupled to the second interconnecting member. In another aspect, the first interconnecting member and the second interconnecting member are sutures. In another aspect, the first interconnecting member and the second interconnecting member are materials having an overall width approximating that of a surgical suture.

In another aspect, an implantable device for anatomical support includes an anatomical support member and an interconnecting member that is coupled to the anatomical support member. An adjustable anchor is slidably coupled to the interconnecting member to permit bi-directional movement along the interconnecting member and configured to exert a compressive force generating frictional interference between the adjustable anchor and the interconnecting member, to inhibit the bi-directional movement of the adjustable anchor along the interconnecting member unless sufficient force is applied to overcome the frictional interference. In another aspect, the anatomical support member is a shaped mesh material for treatment of prolapse. In another aspect, the interconnecting member is a suture. In another aspect, the interconnecting member is a material having an overall width approximating that of a surgical suture.

In another aspect, an implantable device for anatomical support includes an anatomical support member, an interconnecting member that is coupled to the anatomical support member, and an anchor in freely sliding engagement with the interconnecting member. A tensioning element is slidably coupled to the interconnecting member to permit movement along the interconnnecting member and configured to exert a compressive force generating frictional interference between the tensioning element and the interconnecting member, to inhibit the movement of the tensioning element along the interconnecting member unless sufficient force is applied to overcome the frictional interference. In another aspect, the interconnecting member is a suture. In another aspect, the interconnecting member is a material having an overall width approximating that of a surgical suture.

In another aspect an adjustable anchor, for use with an anatomical support member having an interconnecting member extending therefrom, includes a body having a proximal end and a distal end, wherein the distal end includes a flange section that is wider than the proximal end. A collar surrounds, and generates a compressive force against, the proximal end of the body, wherein the interconnecting member is disposed between the body and the collar, subject to the compressive force that generates frictional interference to inhibit bi-directional movement of the adjustable anchor along the interconnecting member unless sufficient force is applied to overcome the frictional interference. In another aspect, a plurality of flanges protrude from the flange section, separated by webs. In another aspect, at least one flange has an angled edge. In another aspect, at least one web is self-creasing.

In another aspect an adjustable anchor and a tool, for placing in a patient an anatomical support member having an interconnecting member extending therefrom, includes an anchor body having a proximal end, a distal end, and a channel extending longitudinally through the anchor body, wherein the distal end includes a flange section that is wider than the proximal end. An anchor collar surrounds, and generates a compressive force against, the proximal end of the anchor body, wherein the interconnecting member is disposed between the anchor body and the anchor collar, subject to the compressive force that generates frictional interference to inhibit bi-directional movement of the adjustable anchor along the interconnecting member unless sufficient force is applied to overcome the frictional interference. A tool shaft has a proximal end, a shoulder, and a distal tip proximate the shoulder. A helical curve in the shaft terminates at the shoulder. The distal tip is configured to be placed in the channel through the anchor body such that the shoulder abuts the anchor body adjacent to the flange section. The helical curve is configured to guide the distal tip from a vaginal incision, around a descending ramus, and through an obturator foremen. In another aspect, a handle is coupled to the proximal end.

In another aspect a surgical method is explained for use with (i) an implantable device having an anatomical support member, a fixed anchor coupled to the implantable device, an adjustable anchor, and an interconnecting member that couples the implantable device to the adjustable anchor in frictional sliding engagement, (ii) a first tool corresponding to a first side of a patient, and (iii) a second tool corresponding to a second side of a patient. The method includes placement of the fixed anchor on a distal tip of the first tool. A vaginal incision in the patient is entered with the fixed anchor on the distal tip of the first tool. The first tool is rotated in a direction corresponding to the first side of the patient such that the fixed anchor travels in a path around a descending pubic ramus on the first side of the patient, continuing in the path until the fixed anchor is placed in obturator tissue on the first side of the patient; and the first tool is removed from the patient. An adjustable anchor is placed on a distal tip of the second tool. The vaginal incision in the patient is entered with the adjustable anchor on the distal tip of the second tool. The second tool is rotated in a direction corresponding to the second side of the patient such that the adjustable anchor travels in a path around a descending pubic ramus on the second side of the patient, continuing in the path until the adjustable anchor is placed in obturator tissue on the second side of the patient; and the second tool is removed from the patient. The interconnecting member, in frictional sliding engagement with the adjustable anchor, is pulled to adjust a length of the interconnecting member between the anatomical support member and the adjustable anchor.

### BRIEF DESCRIPTION OF THE DRAWINGS

Figure 1 is an illustration of one embodiment of an implantable device for anatomical support.
Figure 2 is an exploded illustration of a component of the implantable device shown in Figure 1.
Figure 3 is an exploded illustration of another component of the implatable device shown in Figure 1.
Figure 4 is an assembled, top view of the component shown in Figure 3.
Figure 5 is an illustration of the implantable device shown in Figure 1, after implantation in a patient.
Figure 6 is an illustration of another embodiment of an implantable device for anatomical support.
Figure 7 is a magnified illustration of components of the implantable device shown in Figure 6.
Figure 7A is a cross-sectional view of components shown in Figure 7, taken along lines 7A-7A.
Figure 8 is a magnified illustration of one of the components shown in Figure 6.
Figure 8A is a top view of the component shown in Figure 8.
Figure 9 is a magnified illustration of an alternative component for the device shown in Figure 6.
Figure 9A is a top view of the component shown in Figure 9.
Figure 10A is a partial illustration of another embodiment of an implantable device for anatomical support.
Figure 10B is an illustration of another embodiment of an implantable device for anatomical support.
Figure 10C is an illustration of another embodiment of an implantable device for anatomical support
Figure 11 is an illustration of one embodiment of a pair of tools for use in a surgical method to place an anatomical support member in a patient.
Figure 12 is a magnified, partial illustration of one of the tools shown in Figure 11, coupled to a component shown in Figure 1.

### DETAILED DESCRIPTION

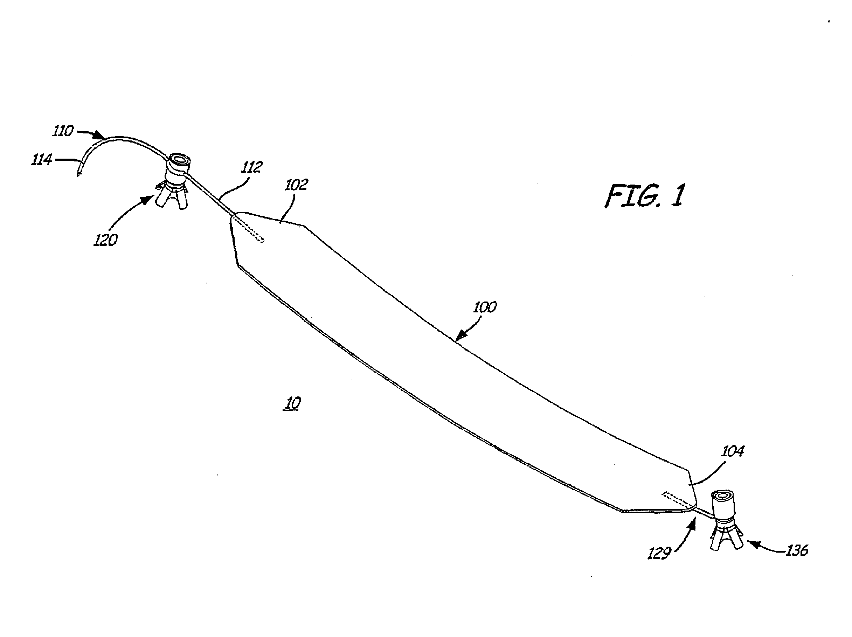

One embodiment of an implantable device for anatomical support (device 10) is illustrated in Figure 1. Therein, an anatomical support member in a form of a suburethral sling includes anchors that are deployed into a patient's tissues. The anchors are coupled to the sling by interconnecting members. In this regard a fixed anchor is fixedly connected in fixed relation to the sling by a first interconnecting member, and an adjustable anchor is slidably coupled in adjustable relation to the sling by a second interconnecting member. The adjustable anchor, as will be described, is configured to permit bidirectional movement along the second interconnecting member in frictional sliding engagement therewith. In one embodiment, the interconnecting members are lengths of suture or suture-like material.

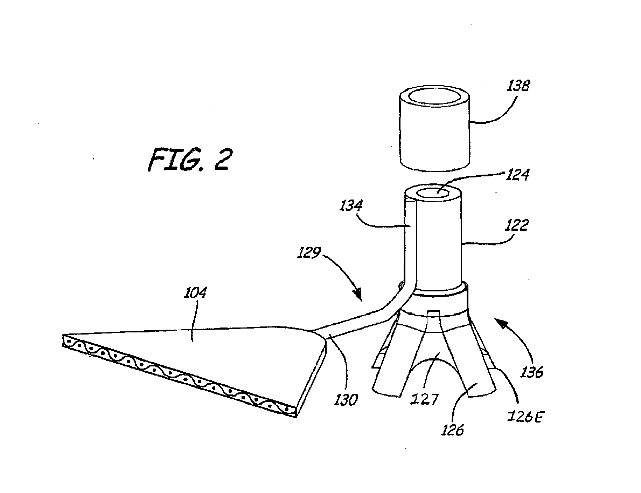

With particular reference to Figures 1 and 2, an example of device 10 includes a suburethral sling 100 with opposing ends 102 and 104. Device 10 also includes interconnecting member 110 having opposing ends 112 and 114, and interconnecting member 129 having opposing ends 130 and 134. End 112 of interconnecting member 110 is coupled to end 102 of sling 100; and as shown in Figure 2 end 130 of interconnecting member 129 is coupled to end 104 of sling 100. Although shown in the drawings via phantom lines as being coupled to an underside or bottom surface of sling 100, it is to be understood that the coupling of interconnecting members 110 and 129 to sling 100 may be provided at any suitable surface cf sling 100 and at any suitable orientation thereon.

Also as shown in Figure 2, in one embodiment device 10 includes a fixed anchor 136 having a body 122 with a proximal end and a distal end, and a channel 124 extending longitudinally therethrough. A plurality of flanges 126 protrude from the distal end, separated by webs 127. End 134 of interconnecting member 129 is fixedly coupled to body 122. Fixed anchor 136 also includes a collar 138. When assembled for use in device 10 as shown in Figure 1, collar 138 covers the proximal end of body 122 of fixed anchor 136 and end 134 of interconnecting member 129 coupled to body 122.

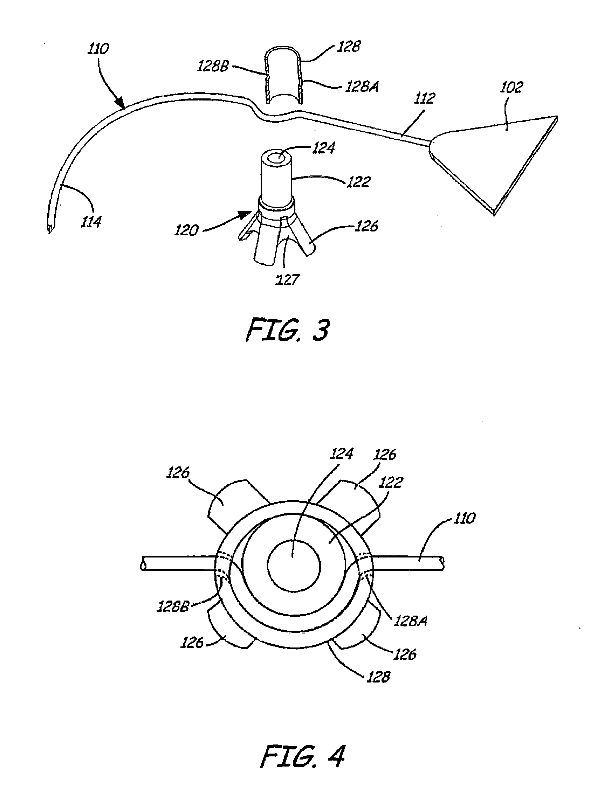

Device 10 also includes an adjustable anchor 120. Referring to Figures 3 and 4, in one embodiment adjustable anchor 120 includes a body 122 having a proximal end and a distal end, with a channel 124 extending longitudinally therethrough and a plurality of flanges 126 protruding from the distal end that are in turn separated by webs 127. As shown in Figure 3 in exploded half-section, and in a top assembly view in Figure 4, adjustable anchor 120 has a collar 128 surrounding the proximal end that includes a pair of apertures 128A and 128B. When assembled for use in device 10, collar 128 covers body 122 of adjustable anchor 120 while apertures 128A-B in collar 128 permit passage of interconnecting member 110 therethrough in frictional sliding engagement with adjustable anchor 120. In this regard and with reference to Figure 4, it is to be appreciated and understood that interconnecting member 110 is disposed through aperture 128A of collar 128, around a partial circumference of body 122, and through aperture 128B of collar 128. By virtue of an intentionally close fit to exert a compressive force and thus frictional interference between interconnecting member 110, collar 128, and body 122, adjustable anchor 120 is slidably coupled to interconnecting member 110 to permit bi-directional movement along interconnecting member 110 upon overcoming such frictional interference.

It is to be understood that an amount of compressive force and thus desired frictional interference could be varied among embodiments of adjustable anchor 120 with regard to an elasticity of a particular material chosen for collar 128 and also with regard to placement of apertures 128A and 128B in collar 128. For example, with locations of apertures 128A-B being constant, if a material chosen for collar 128 in a first embodiment of adjustable anchor 120 has less elasticity than a material chosen for collar 128 in a second embodiment of adjustable anchor 120, then the compressive force and resulting frictional interference of the first embodiment would be greater than that of the second embodiment due to, comparatively, greater resistance of collar 128 against interconnecting member 110 in the first embodiment than in the second embodiment. Similarly, with a material for collar 128 being constant, if apertures 128A-B are placed farther apart in one embodiment of anchor 120 than in a second embodiment of anchor 120, then the compressive force and resulting frictional interference of the first embodiment would be greater than that of the second embodiment due to, comparatively, a longer path through adjustable anchor 120 of interconnecting member 110 in the first embodiment than in the second embodiment.

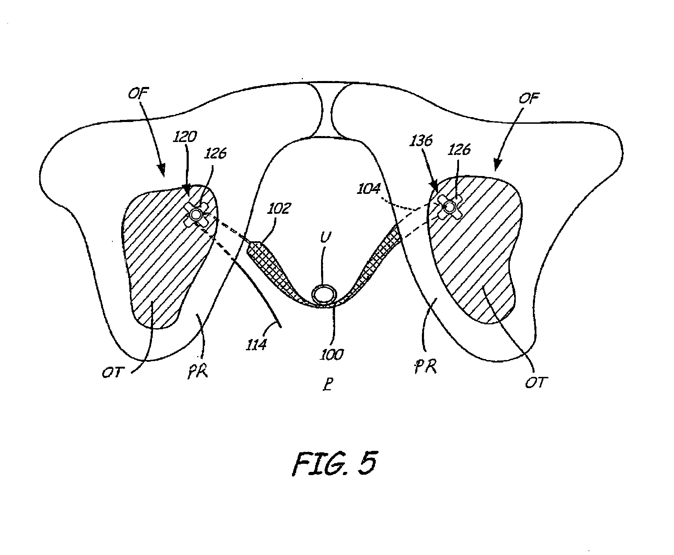

This feature of frictional sliding engagement between interconnecting member 110 and adjustable anchor 120 enables adjustment and tensioning of sling 100 when implanted in a patient. Referring to Figure 5, one embodiment of device 10 is illustrated as having been implanted in a pelvic region P of a patient that includes urethra U and obturator tissue OT in each obturator foramen OF. In the drawing suburethral sling 100 of device 10 is shown as having been positioned under the patient's urethra U, with placement of fixed anchor 136 in obturator tissue OT of one obturator foramen OF and placement of adjustable anchor 120 in obturator tissue OT in the other obturator foramen OF. If desired, positions of anchors 120 and 136 could be exchanged in a left and right sense relative to pelvic region P. As will be further described, flanges 126 and webs 127 of anchors 120 and 136 secure the placement of each anchor in respective obturator tissue OT; and in one embodiment, at least one flange 126 has an angled or beveled edge 126E to promote such secure placement in obturator tissue OT or other anatomical tissue.

In one embodiment, at least one web 127 is self-creasing. Specifically, upon application of pressure to flange 126 such as when anchors 120 and 136 are being deployed through and secured at selected anatomical tissue, web 127 tends to fold or crease which thereby tends to facilitate, advantageously, a temporary bending or deflection of an adjacent flange 126 downwardly and inwardly toward longitudinal channel 124. In turn, this downward or inward bending or deflection of flange 126 tends to facilitate such deployment of the anchor through and into the tissue. Furthermore, upon such deployment through tissue, web 127 advantageously tends to inhibit an inverse bending or deflection of flange 126 upwardly toward body 122.

By way of the coupling of interconnecting members 110 and 129 to anchors 120 and 136 respectively, and the coupling of interconnecting members 110 and 129 to ends 102 and 104 of sling 100 respectively, sling 100 is maintained in position as desired under urethra U. With fixed anchor 136 and adjustable anchor 120 so implanted in obturator tissue OT, and with regard to the frictional sliding engagement between interconnecting member 110 and adjustable anchor 120, it is to be particularly understood that pulling on end 114 of interconnecting member 110 away from adjustable anchor 120 with a force sufficient to overcome the aforementioned interference force between interconnecting member 110 and adjustable anchor 120 would cause interconnecting member 110 to pass through anchor 120 with a resultant shortening of a distance between end 102 of sling 100 and adjustable anchor 120. Thereby, sling 100 would be raised or elevated under urethra U as may be desired and as will be further described. Conversely, pulling on end 112 of interconnecting member 110 away from adjustable anchor 120 (or pulling on sling 100 away from anchor 120, or so pulling on both end 112 and sling 100) with such force would overcome the interference and cause interconnecting member 110 to pass in an opposite direction through anchor 120 with a resultant lengthening of a distance between end 102 of sling 100 and adjustable anchor 120. Thereby, sling 100 would be lowered under urethra U as may be desired and as will be further described.

It is to be appreciated and understood that the novel construction and operation of device 10 is to be provided with respect to three force parameters. First, device 10 is to be constructed such that adjustable anchor 120 is not destroyed or otherwise damaged upon frictional sliding movement of interconnecting member 110 through anchor 120. Second, device 10 is to be constructed such that neither fixed anchor 136 nor, particularly, adjustable anchor 120 are pulled out or dislodged from obturator tissue OT into which they have been placed and secured, upon movement of interconnecting member 110 through adjustable anchor 120 during intraoperative adjustment. Third, device 10 is to be constructed such that the aforementioned interference force between interconnecting member 110 and adjustable anchor 120 is sufficiently high to inhibit movement of sling 100 under urethra U during a provocative event such as coughing by the patient when internal anatomical forces are exerted upon device 10.

In one embodiment, sling 100 has a length of about 7 cm (2.76 in.) and a width in a range of about of 8 mm (0.315 in.) to 11 mm (0.433 in.). Further, in one embodiment sling 100 is a medical grade material such as, for example, knitted polypropylene ARIS ® brand mesh material that is commercially available from Coloplast A/S; and interconnecting members 110 and 129 are lengths of medical grade suture or suture-like materials as aforementioned. In another embodiment, interconnecting members 110 and 129 could be, for example, the aforementioned polypropylene material of sling 100 that has been knitted, woven, or otherwise formed into an elongated suture-like filamentary material. In another embodiment interconnecting members 110 and 129 could be, variously alone or together, continuations of the material of sling 100 configured to have characteristics of a suture-like filamentary material. Accordingly, such embodiments would provide a material having an overall width approximating that of a surgical suture.

Anchors 120 and 136 could be manufactured using any suitable materials such as polypropylene and polyurethane, and fabrication techniques such as molding and milling. In one embodiment, body 122, flanges 126, and webs 127 are fabricated from polypropylene. In one embodiment, collar 128 is molded from a thermoplastic polyurethane material or polymeric elastomer such as TECOTHANE® brand material. In one embodiment, anchors 120 and 136 have an overall length of 0.622 cm (0.245 in.) and a maximum width at flanges 126 of 0.470 cm (0.185 in.). In one embodiment, flanges 126 have a width of 0.114 cm (0.045 in.) and a thickness of 0.038 cm (0.015 in.). In one embodiment, webs 127 have a thickness of approximately one-half that of flanges 126, or about 0.019 cm (0.008 in.). In one embodiment, body 122 has a length of 0.312 cm (0.123 in.) and a diameter of 0.172 cm (0.068 in.). In one embodiment, longitudinal channel 124 in body 122 has a diameter of 0.097 cm (0.038 in.). In one embodiment, before being assembled as described below, collar 128 has an inner diameter of 0.127 cm (0.050 in.), an outer diameter of 0.254 cm (0.100 in.), and a length of 0.318 cm (0.125 in.); and apertures 128A-B have a diameter of 0.051 cm (0.020 in.). In one embodiment, collar 138 of anchor 136 has an inner diameter of 0.191 cm (0.075 in.), an outer diameter of 0.254 cm (0.100 in.), and a length of 0.254 cm (0.100 in.).

In one example of construction of device 10, with reference again to Figure 2, end 112 of interconnecting member 110 is sonically welded to end 102 of sling 100; and end 134 of interconnecting member 129 is sonically welded to end 104 of sling 100. Further in this example, end 134 of interconnecting member 129 is placed against body 122 of anchor 136, and collar 138 is placed over body 122 and end 134. Those assembled components are then sonically welded, thereby securing interconnecting member 129 to anchor 136.

Regarding assembly of adjustable anchor 120, in one embodiment collar 128 is swelled by using a suitable solvent such as methylethylketone (or MEK; also referred to as butanone). Collar 128, manufactured from the thermoplastic polyurethane material as aforementioned, is immersed in the MEK for approximately four hours whereupon it swells or becomes enlarged due to infiltration of the MEK into a molecular composition of the polyurethane material causing its expansion in all dimensions. Swelled collar 128 is then loosely placed over body 122 of adjustable anchor 120, and as aforementioned end 114 of interconnecting member 110 is then passed through aperture 128A of collar 128, around a partial circumference of body 122, and through aperture 128B such that a segment of interconnecting member 110 is within apertures 128A-B. In another embodiment interconnecting member 110 is placed through apertures 128A and 128B of swelled collar 128 such that a segment of interconnecting member 110 is within apertures 128A-B, and then collar 128 is placed over body 122 of adjustable anchor 120. That assembly is then raised to a temperature of 30C for approximately 24 hours, to accelerate evaporation of the MEK from the thermoplastic polyurethane material. When the MEK evaporates, the swelling of collar 128 decreases, effectively returning collar 128 to its pre-swelled dimensions. Thereby, collar 128 tightly surrounds body 122 and interconnecting member 110 disposed therebetween. A result of such assembly is that interconnecting member 110 is movable through apertures 128A-B of collar 128, in frictional sliding contact between body 122 and an inside surface of collar 128.

Although a path through apertures 128A-B is illustrated as being perpendicular to longitudinal channel 124, one aperture 128A or 128B could be at a higher or lower point on collar 128 than the other aperture and thus the path through apertures 128A-B could be at another angle relative to channel 124.

Also, it is to be understood that the aforedescribed connections of components by sonic welding could instead be accomplished by any other suitable means such as, for example, by use of a suitable adhesive material.

In another embodiment, anchor 136 could be coupled directly to anatomical support member 100. In such an embodiment, interconnecting member 129 could be omitted and end 104 could be, for example, sonically welded, glued, or otherwise mechanically coupled to anchor 136 between an outside surface of body 122 and an inside surface of collar 128. In another embodiment, collar 128 could be omitted with, simply, connection of end 104 to body 122.

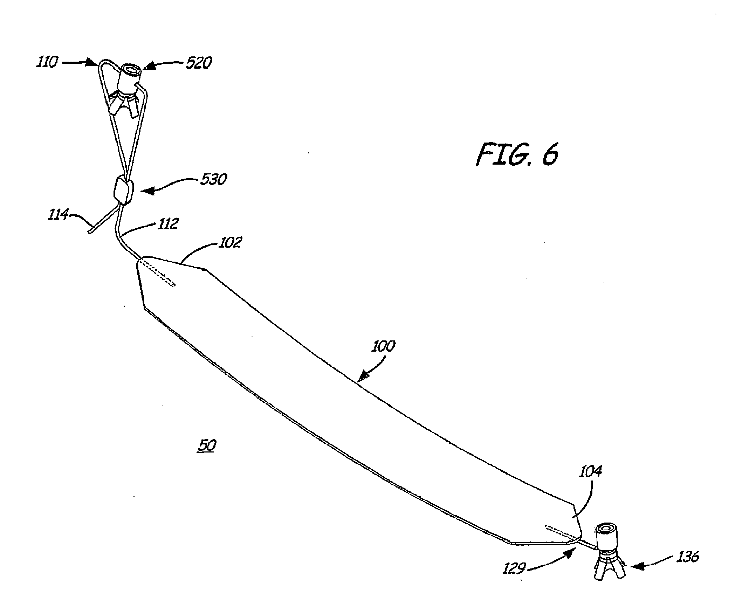

Illustrated in Figure 6 is another example of an implantable device for anatomical support (device 50). In the drawings, like reference numerals denote like components among embodiments. Example device 50 includes an anatomical support member as a suburethral sling 100 with ends 102 and 104; interconnecting member 110 with ends 112 and 114; and interconnecting member 129 with ends 130 and 134. End 112 of interconnecting member 110 is coupled to end 102 of sling 100; and end 130 of interconnecting member 129 is fixedly coupled to end 104 of sling 100. Although shown in the drawings via phantom lines as being coupled to an underside or bottom surface of sling 100, it is to be understood that the coupling of interconnecting members 110 and 129 to sling 100 may be provided at any suitable surface of sling 100 and at any suitable orientation thereon.

Fixed anchor 136 includes a body 122 having a proximal end and a distal end, with a longitudinal channel 124 extending therethrough. A plurality of flanges 126 protruding from the distal end of body 122, separated by webs 127. End 134 of interconnecting member 129 is fixedly coupled to body 122 of fixed anchor 136; and fixed anchor 136 includes a collar 138. Collar 138 covers the proximal end of body 122 and end 134 of interconnecting member 129 coupled to body 122.

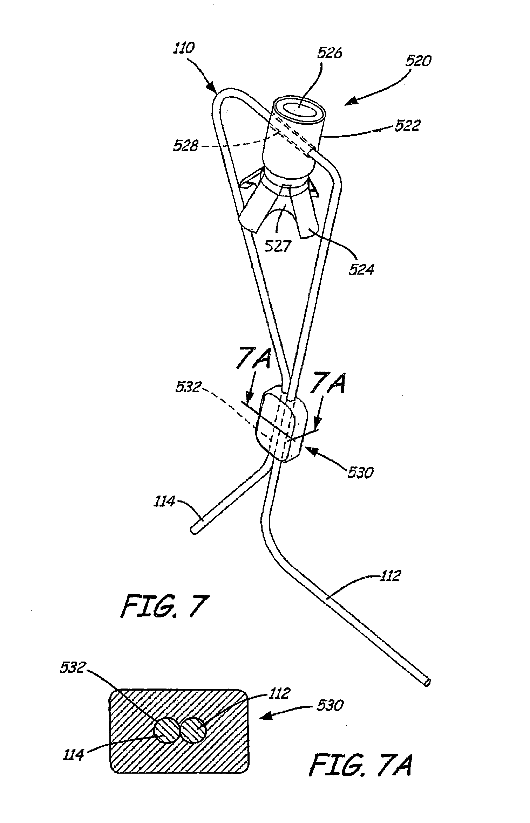

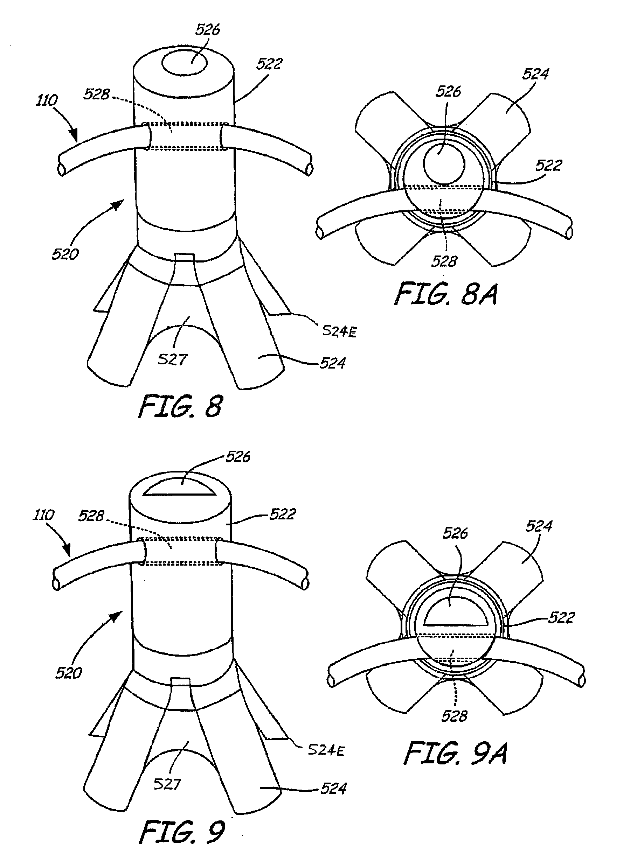

Referring to Figures 7, 8 and 8A, device 50 also includes an anchor 520 and a separate tensioning element 530 slidably coupled to interconnecting member 110. In one embodiment, anchor 520 includes a body 522 having a channel 526 extending longitudinally therethrough, and a plurality of flanges 524 protruding therefrom separated by webs 527; and in one embodiment, at least one flange 524 has an angled or beveled edge 524E to promote secure placement in obturator tissue OT or other anatomical tissue.

In one embodiment, at least one web 527 is self-creasing. Specifically, upon application of pressure to flange 524 such as when anchor 520 is being deployed through and secured at selected anatomical tissue, web 527 tends to fold or crease which thereby tends to facilitate, advantageously, a temporary bending or deflection of an adjacent flange 524 downwardly and inwardly toward longitudinal channel 526. In turn, this downward or inward bending or deflection of flange 524 tends to facilitate such deployment of the anchor through and into the tissue. Furthermore, upon such deployment through tissue, web 527 advantageously tends to inhibit an inverse bending or deflection of flange 524 upwardly toward body 522.

Anchor 520 also has a channel 528 through body 522 to permit interconnecting member 110 to move therethrough in freely sliding engagement with anchor 520. In this example of device 50, and referring to Figures 6, 7, and 7A, interconnecting member 110 is partially disposed within tensioning element 530. In one embodiment, tensioning element 530 is fabricated from a suitable biocompatible material such as, e.g., silicone or a low durometer thermoplastic material like polyurethane. In assembly of device 50, ends 112 and 114 of interconnecting member 110 are disposed within tensioning element 530 (indicated by paths 532 in Figure 7). In particular, although not illustrated, it is to be understood that in one embodiment end 114 of interconnecting member 110 is driven through tensioning element 530 by use of, e.g., a needle. End 114 is then placed through channel 528 of anchor 520 and then driven by the needle back through tensioning element 530. As shown in Figure 7A, by virtue of exertion of a compressive force and thus frictional interference between tensioning element 530 and interconnecting member 110, tensioning element 530 is slidably coupled to interconnecting member 110 to permit bi-directional movement along interconnecting member 110 upon overcoming such frictional interference. This feature of sliding frictional interference between interconnecting member 110 and tensioning element 530 permits adjustment and tensioning of sling 100 when implanted in a patient. With reference to Figure 5, it is to be understood that device 50 could be substituted for device 10 and implanted in a pelvic region P of a patient that includes urethra U and obturator tissue OT in each obturator foramen OF. Thus, suburethral sling 100 of device 50 could be positioned under the patient's urethra U, with secure placement of fixed anchor 136 in obturator tissue OT of one obturator foramen OF and by secure placement of anchor 520 in obturator tissue OT in the other obturator foramen OF. Positions of anchors 520 and 136 could be exchanged in a left and right sense relative to pelvic region P. By grasping tensioning element 530 and pulling on end 114 away from tensioning element 530 with a force sufficient to overcome the aforementioned frictional interference force between interconnecting member 110 and tensioning element 530, interconnecting member 110 slides through tensioning element 530 and thus through anchor 520 with a resultant shortening of a distance between end 102 of sling 100 and tensioning element 530. Thereby, sling 100 would be raised or elevated under urethra U. Conversely, grasping tensioning element 530 and pulling on end 112 of interconnecting member 110 away from tensioning element 530 (or pulling on sling 100 away from tensioning element 530, or so pulling on both end 112 and sling 100) with such force would overcome the interference and cause interconnecting member 110 to pass through tensioning element 530 and thus in an opposite direction through tensioning element 530 with a resultant lengthening of a distance between end 102 of sling 100 and tensioning element 530. Thereby, sling 100 would be lowered under urethra U.

Like device 10, it is to be appreciated and understood that the novel construction and operation of device 50 is to be provided with respect to three force parameters. First, device 50 is to be constructed such that tensioning element 530 is not destroyed or otherwise damaged upon frictional sliding movement of interconnecting member 110 through it. Second, device 50 is to be constructed such that neither anchor 136 nor anchor 520 are pulled out or dislodged from obturator tissue OT into which they have been placed and secured, upon of movement of interconnecting member 110 through tensioning element 530 during intraoperative adjustment. Third, device 50 is to be constructed such that the aforementioned interference force between interconnecting member 110 and tensioning element 530 is sufficiently high to inhibit movement of sling 100 under urethra U during a provocative event when the patient's internal anatomical structures or tissues exert forces upon device 50.

In one embodiment of device 50, components of anchor 520 could be constructed in dimensions, and from materials and techniques, as variously described regarding similar components of fixed anchor 136 in device 10. Furthermore, components of one embodiment of device 50 could be coupled and secured as described relative to similar components of device 10.

Another embodiment of anchor 520 is depicted in Figures 9 and 9A wherein channel 526 is a generally semi-circular or "D" shape. D-shaped channel 526, extending longitudinally through body 522, could provide more clearance for channel 528 compared to the longitudinal and fully cylindrical channel 526 shown in Figures 7, 8 and 8A. Furthermore, and although not illustrated, longitudinal channel 526 could also be provided in a smaller diameter than as shown in Figures 8A and 9A to thereby provide even greater clearance for channel 528. A path through channel 528 is illustrated as being perpendicular to longitudinal channel 526; but in another embodiment, the path could be at another angle relative to channel 526.

It is to be appreciated that when implanted in a patient, sling 100 of devices 10 and 50 advantageously extends nearly from obturator tissue OT on one side of the patient to obturator tissue OT on an opposite side of the patient as a result of, e.g., an intentionally short segment of interconnecting member 129 that couples end 104 of sling 100 to fixed anchor 135 and a selected length of sling 100 with respect to a typical distance between opposing obturator foramen OF.

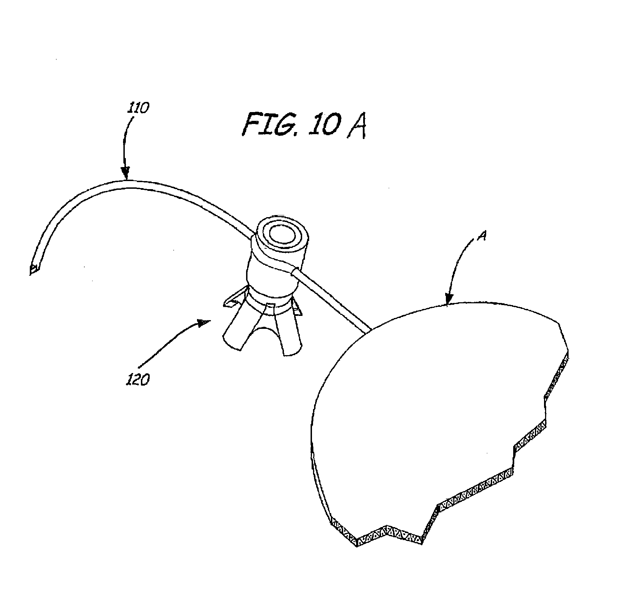

Referring to Figure 10A, and with additional reference to Figures 1, 3, and 4, it is to be appreciated that the novel adjustable anchor 120 described herein could be useful for secure placement of virtually any anatomical support member (A) coupled to an interconnecting member 110 where it is desired to provide adjustment or tensioning of the support member when implanted in a patient. Anatomical support member (A) could be, for example, a shaped mesh material for treatment of prolapse. Also, an anatomical support member could employ any number of adjustable anchors 120, with or without any number of fixed anchors 136.

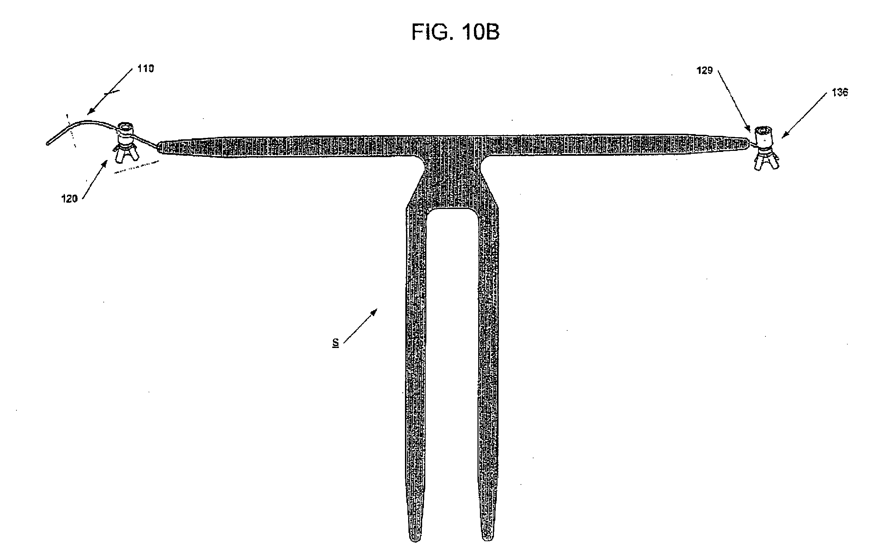

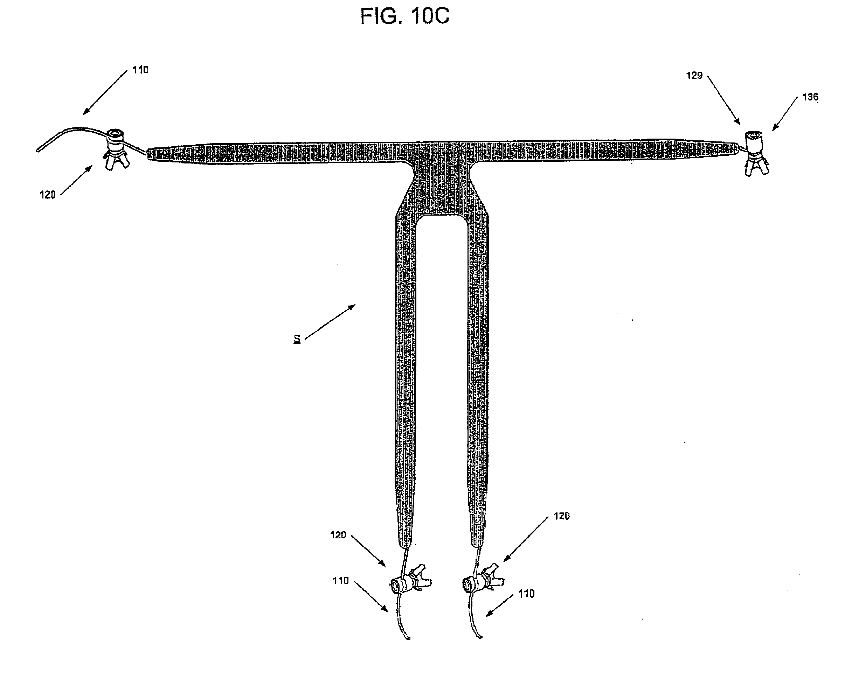

Referring to Figures 10B and 10C, it is to be also appreciated that the novel adjustable anchor 120 described herein could be useful with an implantable device (S) for treatment of urinary incontinence where it is desired to provide adjustment or tensioning of device (S) when implanted in a patient. Although not specifically depicted in Figures 10B-C, it is to be understood however that device (S) could employ any number of adjustable anchors 120, with or without any number of fixed anchors 136.

Although not illustrated in Figures 10A-C, it is to be understood that anchor 520 with tensioning element 530 could be utilized with any anatomical support member (A); and any number of combinations of anchor 520 with tensioning element 530 could also be utilized with or without any number of fixed anchors 136.

Regardless of a particular embodiment of adjustable anchor 120, or of anchor 520 with tensioning element 530, it is to be understood and appreciated that such novel anchors described herein may be relatively small when compared to known anatomical anchors. This advantage results from the fact that the novel anchors described herein are coupled to anatomical support members by sutures or suture-like filaments, rather than directly to the anatomical support members themselves which are usually larger and wider than sutures or suture-like filaments as in some known anatomical anchors. In alternative embodiments, any of the anchors (e.g., anchors 120, 136, or 520) would include at least one flange 126.

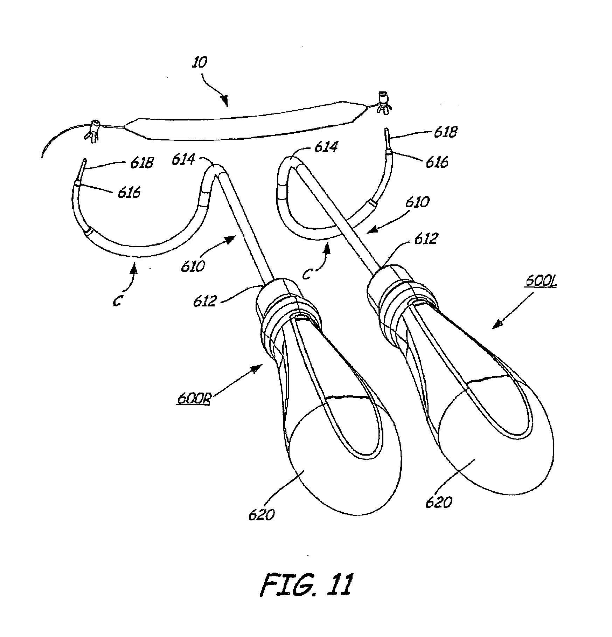

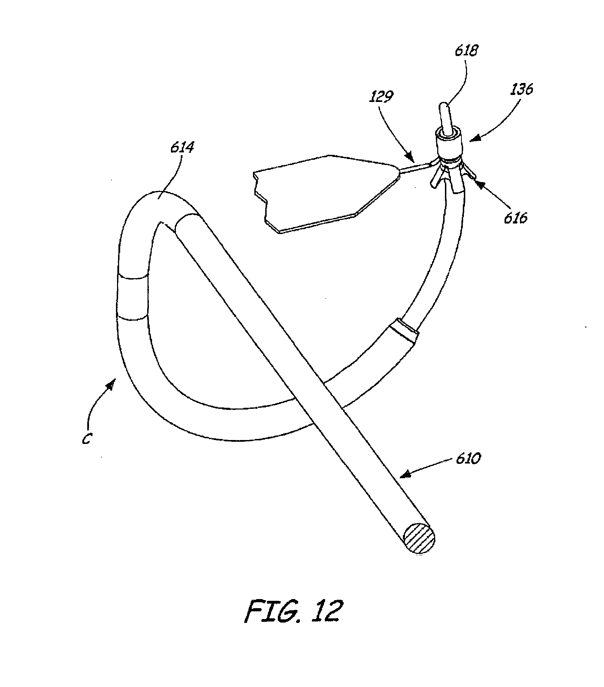

Figures 11 and 12 illustrate an example of a tool for use in placing an implantable device for anatomical support in a patient, such as sling 100 of Figure 1. In the drawing, a pair of tools 600R and 600L are illustrated, in left hand and right hand embodiments - with such designation referring to a patient's left and right sides, respectively. It is to be understood that the tools are identical except for a direction of a helical curve C as described below.

In this example, tools 600R and 600L each include a shaft 610 having a proximal end 612 and a cylindrical distal tip 618. A handle 620 is coupled to proximal end 612 of shaft 610. Handle 620 could have any desired shape or configuration with respect to ergonomic and other considerations of interest. A generally helical curve C is provided in shaft 610. Helical curve C terminates in a shoulder 616 proximate to distal tip 618. In use as described below, helical curve C is advantageously configured to guide tip 618 from an incision (e.g., a vaginal incision in a female patient or a perineal incision in a male patient), around a descending ramus, and through an obturator foramen OF in the patient. In this example, and as shown in Figure 12, cylindrical distal tip 618 is configured to be placed through cylindrical channels 124 of adjustable anchor 120 and fixed anchor 136 (as shown in, e.g., in Figures 2 and 3), and through cylindrical chancel 526 of anchor 520 (as shown, e.g., in Figures 7, 8, and 8A). When so placed, shoulder 616 abuts the anchor's body adjacent to the flanges with the anchor being thereby carried on tip 618 of tool 600R or 600L. Although not illustrated, it is to be understood that if an anchor was constructed with a semi-circular or "D" shaped channel 526 as depicted in Figures 9 and 9A, tip 618 would then be a complementary semi-circular or "D" shaped configuration.

In one embodiment, handle 620 has a length of 11.43 cm (4.5 in.). A length of shaft 610, from handle 620 to a beginning point 614 of curve C is 17.78 cm (7.0 in.). Shaft 610 has a diameter of 3 mm (0.12 in.) decreasing to 1 mm (0.04 in.) at shoulder portion 616. Curve C has a radius of curvature in a range of 2.03 cm (0.80 in.) to 2.54 cm (1.0 in.). Suitable materials for construction of handle 620 include, for example, a medical grade thermoplastic or thermoset material, preferably having both high and low durometer regions for ergonomic considerations. A suitable material for construction of shaft 610 is, for example, medical grade stainless steel. Furthermore, the tool described herein - such as the examples of tools 600R and 600L - could be disposable or sterilizable and reusable.

It is to be appreciated that in one embodiment, as shown particularly in Figure 12, a length of distal tip 618 is chosen so that it protrudes from an anchor seated on shoulder 616. When constructed from stainless steel as aforementioned, relatively stiff tip 618 is thereby configured to pierce anatomical tissue when in use as described below. Thereby, the anchor itself does not need to include such a tissue-penetrating tip.

Referring in particular to Figures 1, 5, 11, and 12, an example of a surgical method to implant a device for anatomical support 10, in a form of suburethral sling 100 for treatment of urinary incontinence in a female patient, is as follows.

A catheter is placed in the patient's urethra U, among other usual and preliminary steps in preparation for surgery. The patient is placed on an operating table in a slightly exaggerated lithotomy position with buttocks extending just beyond an edge of the table. With the patient under anesthesia, a vaginal incision and blunt dissection are made. In one example of the method, a fixed anchor is first placed in obturator tissue OT on the patient's left side, followed by placement of an adjustable anchor in obturator tissue OT on the patient's right side. Accordingly in this embodiment, fixed anchor 136 is placed on distal tip 618 of left hand tool 600L having an orientation of helical curve C corresponding to the patient's left side. Tip 618 of left hand tool 600L, with fixed anchor 136 seated thereupon, is placed within the vaginal incision. Left hand tool 600L is then rotated such that rotation of helical curve C advances tip 618 and fixed anchor 136 in a path around a descending pubic ramus (PR) on the patient's left side, continuing in that path until fixed anchor 136 penetrates obturator tissue OT on the patient's left side (as may be indicated by an audible or tactile "pop") and is thus secured therein. By virtue of flanges 126, fixed anchor 136 is inhibited from being pulled back through obturator tissue OT so penetrated as shown in Figure 5. Left hand tool 600L is then removed from the patient. Next in this example, adjustable anchor 120 is placed on distal tip 618 of right hand tool 600R having an orientation of helical curve C corresponding to the patient's right side. Tip 618 of right hand tool 600R, with adjustable anchor 120 seated thereupon, is placed within the vaginal incision. Right hand tool 600R is then rotated such that rotation of helical curve C advances tip 618 and adjustable anchor 120 in a path around a descending pubic ramus (PR) on the patient's right side, continuing in that path until adjustable anchor 120 penetrates obturator tissue OT on the patient's right side (as may be indicated by an audible or tactile "pop") and is thus secured therein. By virtue of flanges 126, adjustable anchor 120 is inhibited from being pulled back through obturator tissue OT so penetrated as shown in Figure 5. Right hand tool 600R is then removed from the patient.

With suburethral sling 100 thus placed and secured in the patient by way of fixed anchor 136 and adjustable anchor 120, an assessment is made of whether sling 100 is unacceptably loose or tight under urethra U. If sling 100 is unacceptably loose, then end 114 of interconnecting member 110 is pulled away from adjustable anchor 120 with a force sufficient to overcome the aforementioned interference force between interconnecting member 110 and adjustable anchor 120. Interconnecting member 110 thus passes through anchor 120 with a resultant shortening of a distance between end 102 of sling 100 and adjustable anchor 120. Thereby sling 100 is raised or elevated under urethra U as desired. Conversely, if sling 100 is unacceptably tight, then end 112 of interconnecting member 110 is pulled away from adjustable anchor 120 (or sling 100 is pulled away from adjustable anchor 120, or both end 112 and sling 100 are so pulled) with a force sufficient to overcome the interference force between interconnecting member 110 and adjustable anchor 120. Interconnecting member 110 thus passes through anchor 120 with a resultant lengthening of a distance between end 102 of sling 100 and adjustable anchor 120. Thereby sling 100 is lowered under urethra U as desired. These steps of shortening and lengthening a distance between end 102 of sling 100 and adjustable anchor 120 may be repeated in any order and as frequently as necessary to provide optimal suburethral support from sling 100 to urethra U. The vaginal incision is then closed and usual post-operative procedures are performed.

In another example, the aforedescribed method could employ an example of device 50 as shown in Figures 6-8A. In this example of the method, a catheter is placed in the patient's urethra U and the aforementioned preliminary steps in preparation for surgery are performed. The patient is placed in a slightly exaggerated lithotomy position with buttocks extending just beyond an edge of an operating table; and under anesthesia, a vaginal incision and blunt dissection are made in the patient. In one example of this method using device 50, a fixed anchor is first placed in obturator tissue OT on the patient's left side, followed by placement of an anchor in obturator tissue OT on the patient's right side that is associated with a separate tensioning element. Accordingly, fixed anchor 136 is placed on distal tip 618 of left hand tool 600L having an orientation of helical curve C corresponding to the patient's left side. Tip 618 of left hand tool 600L, with fixed anchor 136 seated thereupon, is placed within the vaginal incision. Left hand tool 600L is then rotated such that rotation of helical curve C advances tip 618 and fixed anchor 136 in a path around a descending pubic ramus (PR) on the patient's left side, continuing in that path until fixed anchor 136 penetrates obturator tissue OT on the patient's left side (as may be indicated by an audible or tactile "pop") and is thus secured therein. By virtue of flanges 126, fixed anchor 136 is inhibited from being pulled back through obturator tissue OT so penetrated as shown in Figure 5. Left hand tool 600L is then removed from the patient. Next in this example using device 50, anchor 520 is placed on distal tip 618 of right hand tool 600R having an orientation of helical curve C corresponding to the patient's right side. Tip 618 of right hand tool 600R, with anchor 520 seated thereupon, is placed within the vaginal incision. Right hand tool 600R is then rotated such that rotation of helical curve C advances tip 618 and anchor 520 in a path around a descending pubic ramus (PR) on the patient's right side, continuing in that path until anchor 520 penetrates obturator tissue OT on the patient's right side (as may be indicated by an audible or tactile "pop") and is thus secured therein. By virtue of flanges 126, anchor 520 is inhibited from being pulled back through obturator tissue OT so penetrated. Right hand tool 600R is then removed from the patient.

With suburethral sling 100 of device 50 thus placed and secured in the patient by way of fixed anchor 136 and anchor 520, an assessment is made of whether sling 100 is unacceptably loose or tight under urethra U. If sling 100 is unacceptably loose, then tensioning element 530 is grasped and end 114 of interconnecting member 110 is pulled away from tensioning element 530 with a force sufficient to overcome the aforementioned interference force between interconnecting member 110 and tensioning element 530. Interconnecting member 110 thus passes through anchor 520 with a resultant shortening of a distance between end 102 of sling 100 and tensioning element 530. Thereby sling 100 is raised or elevated under urethra U as desired. Conversely, if sling 100 is unacceptably tight, then tensioning element 530 is grasped and end 112 of interconnecting member 110 is pulled away from tensioning element 530 (or sling 100 is pulled away from tensioning element 530, or both end 112 and sling 100 are so pulled) with a force sufficient to overcome the interference force between interconnecting member 110 and tensioning element 530. Interconnecting member 110 thus passes through anchor 120 with a resultant lengthening of a distance between end 102 of sling 100 and tensioning element 530. Thereby sling 100 is lowered under urethra U as desired. Similarly to device 10, these steps of shortening and lengthening a distance between end 102 of sling 100 and tensioning element 530 in device 50 may be repeated in any order and as frequently as necessary to provide optimal suburethral support from sling 100 to urethra U. The vaginal incision is then closed and usual post-operative procedures are performed.

It is to be again appreciated that components of these devices could be reversed, if desired, in a right side / left side sense from their arrangements as shown in the examples of Figures 1 and 5. It is also to be appreciated that the aforedescribed method steps could be performed in other sequences as may be desired.

It is also to be appreciated that the examples of methods described herein, for surgical placement of devices for anatomical support, do not require skin exits or incisions other than for a single vaginal incision (or, in a male patient, a single perineal incision) for placement and adjustment.

Upon occurrence of tissue in-growth, after implantation surgery is completed and during the patient's healing process, anchors might then become unnecessary to continue to secure the anatomical support device in the patient. Therefore, any of the anchors and the interconnecting members could be made of a suitable medical grade bioresorbable material.

It is to be also appreciated that the foregoing examples of implantable devices for anatomical support provide means for adjustment or tensioning of anatomical support members that are not dependent upon anchor placement. For example, increased tensioning of the devices may be advantageously achieved without a need for advancing anchors more deeply into target tissue in the patient. Also, the aforedescribed frictional sliding engagement between interconnecting member 110 and adjustable anchor 120 - or between interconnecting member 110 and tensioning element 530 - permits novel intraoperative adjustment of the implantable devices for anatomical support disclosed herein. Furthermore adjustable anchor 120, as well as the combination of anchor 520 with tensioning element 530, permits such intra-operative adjustment to be performed as many times as desired during a particular implantation procedure, to achieve optimal device placement, adjustment, and tensioning.

While implantable devices, tools, and methods for anatomical support have been particularly shown and described herein with reference to the accompanying specification and drawings, it will be understood however that other modifications thereto are of course possible; . It should be appreciated that (i) components, dimensions, shapes, and other particulars of the example embodiments herein may be substituted for others that are suitable for achieving desired results, (ii) various additions or deletions may be made thereto, and (iii) features of the foregoing examples may also be made in combinations thereof. It is also to be understood in general that any suitable alternatives may be employed to provide these implantable devices, tools, and methods for anatomical support.

Lastly, choices of compositions, sizes, and strengths of various aforementioned elements, components, and steps all depend upon intended uses thereof. Accordingly, these and other various changes or modifications in form and detail may also be made.

## Claims

1. An implantable device (10) for anatomical support, comprising:
an anatomical support member (100);
an interconnecting member (110) that is coupled to said anatomical support member (110); and
an adjustable anchor (120), slidably coupled to said interconnecting member (110) to permit bi-directional movement along said interconnecting member (110) and configured to exert a compressive force generating frictional interference between said adjustable anchor (120) and said interconnecting member (110), to inhibit said bi-directional movement of said adjustable anchor (120) along said interconnecting member (110) unless sufficient force is applied to overcome said frictional interference
**characterized in that** the adjustable anchor (120) comprises:
a body (122) having a proximal end and a distal end, wherein said distal end includes a flange section that is wider than said proximal end; and
a collar (128) surrounding, and generating the compressive force against, said proximal end of said body,
wherein the interconnecting member (110) is disposed between said body (122) and said collar (128), by being disposed through a first aperture (128A) of the collar (128), around a partial circumference of the body (122) and through a second aperture (128B) of the collar (128).

2. The implantable device for anatomical support according to claim 1, further comprising:
that the anatomical support member (100) is a sling;
a second interconnecting member (129) that is coupled to said sling;
a fixed anchor (136), fixedly coupled to said second interconnecting member (129).

3. The implantable device for anatomical support according to claim 1 or 2, wherein said anatomical support member (100) is a shaped mesh material for treatment of prolapse.

4. The implantable device for anatomical support according to any one of the claims 1, 2 or 3, wherein said interconnecting members (110, 129) are sutures.

5. The implantable device for anatomical support according to any one of the claims 1, 2 or 3, wherein said interconnecting members (110, 129) are materials having an overall width approximating that of a surgical suture.

6. An adjustable anchor (120), for use with an anatomical support member (100) having an interconnecting member (110) extending therefrom, **characterized in that** the adjustable anchor (120) comprises:
a body (122) having a proximal end and a distal end, wherein said distal end includes a flange section that is wider than said proximal end; and
a collar (128) surrounding, and generating a compressive force against, said proximal end of said body (122),
wherein the adjustable anchor (122) is adapted to allow the interconnecting member (110) to be disposed between said body (122) and said collar (128) by being disposed through a first aperture (128A) of the collar (128), around a partial circumference of the body (122) and through a second aperture (128B) of the collar (128), such that the compressive force that generates frictional interference to inhibit bi-directional movement of said adjustable anchor (120) along said interconnecting member (110) unless sufficient force is applied to overcome said frictional interference.

7. The adjustable anchor of claim 6, wherein a plurality of flanges (126) protrude from said flange section, separated by webs (127).

8. The adjustable anchor of claim 7, wherein at least one flange (126) has an angled edge (126E).

9. The adjustable anchor according to claim 7 or 8, wherein at least one web (127) is self-creasing.

10. An adjustable anchor (120) and a tool (600R, 600L), for placing in a patient an anatomical support member (100) having an interconnecting member (110) extending therefrom, comprising:
an anchor body (122) having a proximal end, a distal end, and a channel (124) extending longitudinally through said anchor body (122), wherein said distal end includes a flange section that is wider than said proximal end;
an anchor collar (128) surrounding, and generating a compressive force against, said proximal end of said anchor body (122),
wherein the interconnecting member (110) is disposed between said anchor body (122) and said anchor collar (128) by being disposed through a first aperture (128A) of the collar (128), around a partial circumference of the body (122) and through a second aperture (128B) of the collar (128), such that the compressive force that generates frictional interference to inhibit bi-directional movement of said adjustable anchor (120) along said interconnecting member (110) unless sufficient force is applied to overcome said frictional interference;
a tool shaft (610) having a proximal end (612), a shoulder (616), and a distal tip (618) proximate said shoulder (616); and a helical curve in said shaft (610), terminating at said shoulder (616),
wherein said distal tip (618) is configured to be placed in said channel (124) through said anchor body (122) such that said shoulder (616) abuts said anchor body (122) adjacent to said flange section, and said helical curve is configured to guide said distal tip (618) from a vaginal incision, around a descending ramus, and through an obturator foramen.

11. The adjustable anchor and tool of claim 10, further comprising a handle (620) coupled to said proximal end (612).

## Patentansprüche

1. Implantierbare Vorrichtung (10) für die anatomische Unterstützung, umfassend:
ein anatomisches Unterstützungselement (100),
ein Verbindungselement (110), das an das anatomische Unterstützungselement (100) gekoppelt ist, und
einen verstellbaren Anker (120), der verschiebbar an das Verbindungselement (110) gekoppelt ist, um eine bidirektionale Bewegung entlang dem Verbindungselement (110) zu gestatten, und dazu konfiguriert ist, eine Druckkraft auszuüben, die Reibungsinterferenz zwischen dem verstellbaren Anker (120) und dem Verbindungselement (110) erzeugt, um die bidirektionale Bewegung des verstellbaren Ankers (120) entlang dem Verbindungselement (110) zu verhindern, es sei denn, es wird hinreichend Kraft ausgeübt, um die Reibungsinterferenz zu überwinden,
**dadurch gekennzeichnet, dass** der verstellbare Anker (120) Folgendes umfasst:
einen Körper (122) mit einem proximalen und einem distalen Ende, wobei das distale Ende einen Flanschabschnitt aufweist, der breiter als das proximale Ende ist, und
einen Bund (128), der das proximale Ende des Körpers umgibt und die Druckkraft dagegen erzeugt,
wobei das Verbindungselement (110) zwischen dem Körper (122) und dem Bund (128) angeordnet ist, indem es durch eine erste Öffnung (128A) des Bunds (128), um einen Teilumfang des Körpers (122) und durch eine zweite Öffnung (128B) des Bunds (128) angeordnet ist.

2. Implantierbare Vorrichtung für die anatomische Unterstützung nach Anspruch 1, ferner umfassend:
dass das anatomische Unterstützungselement (100) eine Schlinge ist,
ein zweites Verbindungselement (129), das an die Schlinge gekoppelt ist, und
einen fixierten Anker (136), der fest an das zweite Verbindungselement (129) gekoppelt ist.

3. Implantierbare Vorrichtung für die anatomische Unterstützung nach Anspruch 1 oder 2, wobei das anatomische Unterstützungselement (100) ein geformtes Netzmaterial zur Prolapsbehandlung ist.

4. Implantierbare Vorrichtung für die anatomische Unterstützung nach einem der Ansprüche 1, 2 oder 3, wobei die Verbindungselemente (110, 129) chirurgisches Nahtmaterial sind.

5. Implantierbare Vorrichtung für die anatomische Unterstützung nach einem der Ansprüche 1, 2 oder 3, wobei die Verbindungselemente (110, 129) Materialien mit einer Gesamtbreite sind, die ungefähr der eines chirurgischen Fadens entspricht.

6. Verstellbarer Anker (120) für den Einsatz mit einem anatomischen Unterstützungselement (100) mit einem sich davon erstreckenden Verbindungselement (110), **dadurch gekennzeichnet, dass** der verstellbare Anker (120) Folgendes umfasst:
einen Körper (122) mit einem proximalen und einem distalen Ende, wobei das distale Ende einen Flanschabschnitt aufweist, der breiter als das proximale Ende ist, und
einen Bund (128), der das proximale Ende des Körpers (122) umgibt und eine Druckkraft dagegen erzeugt,
wobei der verstellbare Anker (120) geeignet ist zu gestatten, dass das Verbindungselement (110) zwischen dem Körper (122) und dem Bund (128) angeordnet wird, indem es durch eine erste Öffnung (128A) des Bunds (128), um einen Teilumfang des Körpers (122) und durch eine zweite Öffnung (128B) des Bunds (128) angeordnet ist, so dass die Druckkraft, die Reibungsinterferenz erzeugt, die bidirektionale Bewegung des verstellbaren Ankers (120) entlang dem Verbindungselement (110) verhindert, es sei denn, es wird hinreichend Kraft ausgeübt, um die Reibungsinterferenz zu überwinden.

7. Verstellbarer Anker nach Anspruch 6, wobei mehrere Flansche (126) von dem Flanschabschnitt vorragen und durch Stege (127) getrennt sind.

8. Verstellbarer Anker nach Anspruch 7, wobei mindestens ein Flansch (126) einen abgewinkelten Rand (126E) hat.

9. Verstellbarer Anker nach Anspruch 7 oder 8, wobei mindestens ein Steg (127) selbstknickend ist.

10. Verstellbarer Anker (120) und Werkzeug (600R, 600L) zum Platzieren eines anatomischen Unterstützungselements (100) mit einem sich davon erstreckenden Verbindungselement (110) in einen Patienten, umfassend:
einen Ankerkörper (122) mit einem proximalen Ende, einem distalen Ende und einem Kanal (124), der sich in Längsrichtung durch den Ankerkörper (122) erstreckt, wobei das distale Ende einen Flanschabschnitt aufweist, der breiter als das proximale Ende ist,
einen Ankerbund (128), der das proximale Ende des Ankerkörpers (122) umgibt und eine Druckkraft dagegen erzeugt,
wobei das Verbindungselement (110) zwischen dem Ankerkörper (122) und dem Ankerbund (128) angeordnet ist, indem es durch eine erste Öffnung (128A) des Bunds (128), um einen Teilumfang des Körpers (122) und durch eine zweite Öffnung (128B) des Bunds (128) angeordnet ist, so dass die Druckkraft, die Reibungsinterferenz erzeugt, die bidirektionale Bewegung des verstellbaren Ankers (120) entlang dem Verbindungselement (110) verhindert, es sei denn, es wird hinreichend Kraft ausgeübt, um die Reibungsinterferenz zu überwinden,
einen Werkzeugschaft (610) mit einem proximalen Ende (612), einer Schulter (616), einer distalen Spitze (618) in der Nähe der Schulter (616) und einer spiralförmigen Biegung in dem Schaft (610), die an der Schulter (616) endet,
wobei die distale Spitze (618) dazu konfiguriert ist, durch den Ankerkörper (122) in den Kanal (124) platziert zu werden, so dass die Schulter (616) neben dem Flanschabschnitt an den Ankerkörper (122) anschlägt, und die spiralförmige Biegung dazu konfiguriert ist, die distale Spitze (618) von einem vaginalen Einschnitt um einen oberen Sitzbeinast und durch ein Hüftloch zu führen.

11. Verstellbarer Anker und Werkzeug nach Anspruch 10, ferner umfassend einen an das proximale Ende (612) gekoppelten Griff (620).

## Revendications

1. Dispositif implantable (10) pour support anatomique, comprenant :
un organe de support anatomique (100) ;
un organe d'interconnexion (110) qui est accouplé audit organe de support anatomique (100) ; et
un ancrage ajustable (120), accouplé de manière coulissante audit organe d'interconnexion (110) pour permettre un mouvement bidirectionnel le long dudit organe d'interconnexion (110) et configuré pour exercer une force de compression générant une interférence de frottement entre ledit ancrage ajustable (120) et ledit organe d'interconnexion (110), pour empêcher ledit mouvement bidirectionnel dudit ancrage ajustable (120) le long dudit organe d'interconnexion (110) à moins qu'une force suffisante soit appliquée pour surmonter ladite interférence de frottement,
**caractérisé en ce que** l'ancrage ajustable (120) comprend :
un corps (122) ayant une extrémité proximale et une extrémité distale,
ladite extrémité distale comportant une section de bride qui est plus large que ladite extrémité proximale ; et
un col (128) entourant, et générant la force de compression contre, ladite extrémité proximale dudit corps,
l'organe d'interconnexion (110) étant disposé entre ledit corps (122) et ledit col (128), en étant disposé à travers une première ouverture (128A) du col (128), autour d'une circonférence partielle du corps (122) et à travers une deuxième ouverture (128B) du col (128).

2. Dispositif implantable pour support anatomique selon la revendication 1, comprenant en outre :
le fait que l'organe de support anatomique (100) est un treillis chirurgical ;
un deuxième organe d'interconnexion (129) qui est accouplé audit treillis chirurgical ;
un ancrage fixe (136), accouplé fixement audit deuxième organe d'interconnexion (129).

3. Dispositif implantable pour support anatomique selon la revendication 1 ou 2, dans lequel ledit organe de support anatomique (100) est un matériau à mailles façonnées pour le traitement du prolapsus.

4. Dispositif implantable pour support anatomique selon l'une quelconque des revendications 1, 2 ou 3, dans lequel lesdits organes d'interconnexion (110, 129) sont des sutures.

5. Dispositif implantable pour support anatomique selon l'une quelconque des revendications 1, 2 ou 3, dans lequel lesdits organes d'interconnexion (110, 129) sont des matériaux ayant une largeur globale approximativement égale à celle d'une suture chirurgicale.

6. Ancrage ajustable (120), destiné à être utilisé avec un organe de support anatomique (100) ayant un organe d'interconnexion (110) s'étendant depuis celui-ci, **caractérisé en ce que** l'ancrage ajustable (120) comprend :
un corps (122) ayant une extrémité proximale et une extrémité distale,
ladite extrémité distale comportant une section de bride qui est plus large que ladite extrémité proximale ; et
un col (128) entourant, et générant une force de compression contre, ladite extrémité proximale dudit corps (122),
l'ancrage ajustable (120) étant prévu pour permettre de disposer l'organe d'interconnexion (110) entre ledit corps (122) et ledit col (128) en étant disposé à travers une première ouverture (128A) du col (128), autour d'une circonférence partielle du corps (122) et à travers une deuxième ouverture (128B) du col (128), de telle sorte que la force de compression qui génère l'interférence de frottement empêche le mouvement bidirectionnel dudit ancrage ajustable (120) le long dudit organe d'interconnexion (110) à moins qu'une force suffisante soit appliquée pour surmonter ladite interférence de frottement.

7. Ancrage ajustable selon la revendication 6, dans lequel une pluralité de brides (126) font saillie depuis ladite section de bride, séparées par des nervures (127).

8. Ancrage ajustable selon la revendication 7, dans lequel au moins une bride (126) a un bord incliné (126E).

9. Ancrage ajustable selon la revendication 7 ou 8, dans lequel au moins une nervure (127) est auto-froissante.

10. Ancrage ajustable (120) et outil (600R, 600L), destinés à placer dans un patient un organe de support anatomique (100) ayant un organe d'interconnexion (110) s'étendant depuis celui-ci, comprenant :
un corps d'ancrage (122) ayant une extrémité proximale, une extrémité distale, et un canal (124) s'étendant longitudinalement à travers ledit corps d'ancrage (122), ladite extrémité distale comportant une section de bride qui est plus large que ladite extrémité proximale ;
un col d'ancrage (128) entourant, et générant une force de compression contre, ladite extrémité proximale dudit corps d'ancrage (122),
l'organe d'interconnexion (110) étant disposé entre ledit corps d'ancrage (122) et ledit col d'ancrage (128) en étant disposé à travers une première ouverture (128A) du col (128), autour d'une circonférence partielle du corps (122) et à travers une deuxième ouverture (128B) du col (128), de telle sorte que la force de compression qui produit l'interférence de frottement empêche le mouvement bidirectionnel dudit ancrage ajustable (120) le long dudit organe d'interconnexion (110) à moins qu'une force suffisante soit appliquée pour surmonter ladite interférence de frottement ;
une tige d'outil (610) ayant une extrémité proximale (612), un épaulement (616), et une pointe distale (618) à proximité dudit épaulement (616) ; et une courbe hélicoïdale dans ladite tige (610), se terminant au niveau dudit épaulement (616),
ladite pointe distale (618) étant configurée pour être placée dans ledit canal (124) à travers ledit corps d'ancrage (122) de telle sorte que ledit épaulement (616) bute contre ledit corps d'ancrage (122) en position adjacente à ladite section de bride, et que ladite courbe hélicoïdale soit configurée pour guider ladite pointe distale (618) depuis une incision vaginale, autour d'une branche descendante et à travers un foramen obturateur.

11. Ancrage ajustable et outil selon la revendication 10, comprenant en outre une poignée (620) accouplée à ladite extrémité proximale (612).
